# EUROPÄISCHE PATENTSCHRIFT

(11) **EP 2 903 539 B1**
(45) Veröffentlichungstag und Bekanntmachung des Hinweises auf die Patenterteilung: **13.06.2018**
(21) Anmeldenummer: 13771461.4
(22) Anmeldetag: 30.09.2013
(51) Int. Cl.: A61B 17/32, A61B 17/3205, A61B 17/3207, A61B 17/00, A61B 17/22

(54) **WEITENVERSTELLBARES SCHNEIDINSTRUMENT ZUR TRANSAPIKALEN AORTENKLAPPENRESEKTION**
WIDTH-ADJUSTABLE CUTTING INSTRUMENT FOR TRANSAPICAL AORTIC VALVE RESECTIONING
INSTRUMENT DE COUPE D'AMPLITUDE RÉGLABLE, DESTINÉ À UNE RÉSECTION DE VALVE AORTIQUE PAR VOIE TRANSAPICALE

(30) Priorität: 04.10.2012 DE 102012109459
(43) Veröffentlichungstag der Anmeldung: 12.08.2015
(73) Patentinhaber: Aesculap AG, 78532 Tuttlingen (DE)
(72) Erfinder: MORALES, Pedro, 78532 Tuttlingen (DE); WEISSHAUPT, Dieter, 78194 Immendingen (DE)
(74) Vertreter: Winter, Brandl, Fürniss, Hübner, Röss, Kaiser, Polte - Partnerschaft mbB
(86) Internationale Anmeldenummer: PCT/EP2013/070363
(87) Internationale Veröffentlichungsnummer: WO 2014/053446

(56) Entgegenhaltungen:
- WO-A1-2013/135792
- DE-A1-102010 009 723
- US-A- 3 667 474
- US-A1- 2005 209 617
- US-A1- 2006 271 081
- US-A1- 2007 185 513
- US-A1- 2008 255 595

## Beschreibung

Die vorliegende Erfindung betrifft ein chirurgisches, insbesondere endoskopisches, Schneidinstrument zur, insbesondere transapikalen, Aortenklappenresektion gemäß dem Oberbegriff des Anspruchs 1.

Bei einer Aortenklappenstenose oder -insuffizienz ist oftmals ein operativer Eingriff indiziert, bei dem die körpereigene Aortenklappe durch eine künstliche Aortenklappe ersetzt wird. Neben der offen-chirugischen Operation, die in Vollnarkose und unter Verwendung einer Herz-Lungen-Maschine erfolgt und bei der das Brustbein aufgesägt, das Herz freigelegt und zum Stillstand gebracht werden muss, kann die Aortenklappe auch in einem minimal-invasiven Verfahren am schlagenden Herzen ersetzt werden. Dabei wird entweder ein Zugangsweg über die Leistenarterie (transfemoral) oder über die Herzspitze (transapikal) gewählt.

Eine mögliche Methode ist das Einbringen einer selbstexpandierenden oder mittels eines Ballons expandierenden Aortenklappenprothese, welche die körpereigene Aortenklappe verdrängt. Jedoch können undefinierte, laterale Öffnungen, welche zwischen der künstlichen Aortenklappe und der Aortenwand entstehen, wenn die natürliche Aortenklappe nicht entfernt wird, sondern durch die künstliche Aortenklappe an die Aortenwandung gequetscht wird, den Blutfluss beeinträchtigen.

Vor diesem Hintergrund empfiehlt es sich, vor dem Einsetzen der künstlichen Aortenklappe zunächst die körpereigene Aortenklappe zu entfernen.

Für die Aortenklappenresektion sind Lösungen bekannt, bei denen die verkalkte Aortenklappe mit Laser herausgeschnitten wird. Es gibt auch Veröffentlichungen, bei denen die verkalkte Aortenklappe im Labor und am Schwein mit einer "Aortenstanze" entfernt wurde.

Das Heraustrennen der verkalkten Aortenklappe mit Laser ist sehr zeitaufwändig und auch nicht sehr präzise. Das Entfernen der Klappe mit einer "Aortenstanze" ist zur Zeit nicht praktikabel, da der starre und bereits auf den Aorteninnendurchmesser abgestimmte Stanzkopf durch die verengte Aortenklappe durchgeführt werden muss und sich dabei die an der Aortenklappe abgelagerten Kalkpartikel lösen und in den Blutkreislauf gelangen können, was zu einer Thrombose führen kann. Ein weiteres Problem dabei ist die Tatsache, dass die Aortendurchmesser im Bereich zwischen ca. 15mm und 35mm variieren, so dass ein starrer Stanzkopf in den meisten Fällen nicht den optimalen Durchmesser besitzt, was wiederum zu Folge hat, dass die darin eingebrachte vorgesehene Aortenklappe keinen optimalen Sitz hat.
Chirurgische Schneidinstrumente sind im Stand der Technik beispielsweise aus den Druckschriften US 2007/185513 A1, auf welcher der Oberbegriff des unabhängigen Anspruchs beruht, US 2005/209617 A1, DE 10 2010 009723 A1, US 2006/271081 A1, US 2008/255595 A1 und US 3 667 474 A bekannt.
Aufgabe der vorliegenden Erfindung ist die Bereitstellung eines chirurgischen Schneidwerkzeugs für eine Aortenklappenresektion, das einen möglichst optimalen Sitz der neuen künstlichen Aortenklappe ermöglicht, ohne dass sich dabei Kalkablagerungen von der Klappe ablösen und in den Blutkreislauf gelangen können. Insbesondere soll ein gegenüber dem Stand der Technik in dieser Hinsicht verbessertes chirurgisches Schneidinstrument zur Aortenklappenresektion bereitgestellt werden.
Diese Aufgabe wird durch die Merkmale des Anspruchs 1 gelöst. Vorteilhafte Weiterbildungen sind Gegenstand der Unteransprüche.
Ein erfindungsgemäßes chirurgisches, insbesondere endoskopisches, Schneidinstrument eignet sich zur, insbesondere transapikalen, Aortenklappenresektion. Es weist eine am distalen Ende eines Werkzeugschafts angeordnete Schneideinheit mit zumindest einem mechanischen Schneidelement zum Durchführen eines zirkulären Schnitts auf. Dieses Schneidelement kann beispielsweise eine ringförmige Schneide aufweisen oder als ein um die Schaftlängsachse drehbare Klinge ausgebildet sein. Erfindungsgemäß ist das zumindest eine Schneidelement über einen Stell- bzw. Spreizmechanismus, insbesondere stufenlos, weitenverstellbar. Insbesondere lässt sich der radiale Abstand des zumindest einen Schneidelements von der Schaftlängsachse bzw. der Schneiddurchmesser an unterschiedliche Aortendurchmesser anpassen.

Durch diese Erfindung wird ein chirurgisches Schneidinstrument bereitgestellt, das im Durchmesser an die anatomischen Gegebenheiten der Aorta, insbesondere dem Aortendurchmesser, genau angepasst werden kann, so dass ein und dasselbe Instrument zur Resektion von Aortenklappen unterschiedlichster Größen bzw. Durchmessern verwendet und jeweils genau angepasst werden kann, anstatt unterschiedliche Instrumente für unterschiedliche Aortendurchmesser herstellen und verwenden zu müssen.

Durch die genaue Anpassung an die jeweiligen Gegebenheiten wird ferner ein optimaler Sitz der neuen künstlichen Aortenklappe erreicht und unter anderem gewährleistet, dass sich die Aortenklappe mit der Aorta sicher verkrallen und somit nicht mehr so einfach migrieren kann. Auch die eigentliche Funktion der Klappe (Durchflussregelung) wird gesteigert, weil sich diese dann möglichst ganz und zirkulär im Situs entfalten kann. Dadurch wird auch der Blutfluss optimiert, weil dieser nicht mehr - wie eingangs geschildert - durch undefinierte, laterale Öffnungen beeinträchtigt wird, wenn die natürliche Aortenklappe entfernt wird.

Gemäß einem Aspekt der Erfindung ist nicht nur das zumindest eine Schneidelement, sondern die Schneideinheit insgesamt, insbesondere stufenlos, weitenverstellbar. Wenn die Schneideinheit nur aus Stellmechanismus und daran angeordneten Schneidelementen gebildet wird und die Außenabmessungen der Schneideinheit lediglich durch den radial bewegbaren Stellmechanismus definiert wird, dann kann durch radiales Bewegen des Stellmechanismus nicht nur der Schneiddurchmesser verändert werden, sondern auch die Schneideinheit insgesamt komprimiert bzw. expandiert werden. Durch diese Weitenverstellung der gesamten Schneideinheit können die Außenabmessungen der Schneideinheit insgesamt soweit reduziert werden, dass diese auch in der Lage ist, durch eine sehr enge (stenotisierte) Aortenklappe durchzutauchen, ohne dass sich dabei Kalkpartikel ablösen, bevor die Schneideinheit oder ein Teil der Schneideinheit anschließend hinter der Aortenklappe auf den Aortendurchmesser aufgeweitet wird.

Am proximalen Werkzeugschaft befindet sich ferner ein Werkzeuggriff zum Halten und Manövieren des Werkzeugschafts und eine Handhabe (Drehknopf) zur manuellen Weitenverstellung des Schneidelements. Auf diese Weise kann das bereits in Schneidfunktionsstellung sich befindende Schneidelement in seinem aktiven (wirksamen) Schneiddurchmesser manuell verstellt werden.
Gemäß einem Aspekt der Erfindung wird die Schneideinheit durch ein um den Werkzeugschaft drehbar gelagertes Schneidwerkzeug mit zumindest einer Schneidklinge gebildet, deren radialer Abstand zur Drehachse bzw. Werkzeugschaftachse, insbesondere stufenlos, eingestellt werden kann und so an unterschiedliche Aortendurchmessern anpassbar ist.
Der radiale Abstand der Schneidklinge kann auf einfache Weise verändert werden, wenn die Schneidklinge an einem Tragarm fest oder lösbar angeordnet ist, der über den Stellmechanismus radial, z.B. nach außen, bewegbar ist. Somit kann die Schneideinheit im zusammengefalteten bzw. komprimierten Zustand in das Herz und durch die natürliche Aortenklappe eingeführt werden und anschließend auf den jeweiligen Aortendurchmesser erweitert bzw. aufgespreizt werden. Gegebenenfalls kann die Schneideinheit nach dem Schneidvorgang wieder verkleinert bzw. zusammengefaltet werden.
Die Schneidklinge kann gemäß einem Aspekt der Erfindung so angeordnet sein, dass sie sich in axialer bzw. zur Werkzeugschaftachse parallelen Richtung erstreckt und in Dreh- bzw. Umfangsrichtung schneidet.
Um zu verhindern, dass die Klinge oder die Klingen beim Einführen des Instruments kein Gewebe versehentlich verletzen, können diese entgegen der Einführrichtung angeordnet sein bzw. zur proximalen Seite des Werkzeugschafts weisen.

Der Stellmechanismus ist dergestalt, dass einer oder mehrere in Umfangsrichtung verteilte Tragarme, welche die Schneidklingen tragen, parallel zur Werkzeugschaftachse ausstellbar und einziehbar sind, so dass die Schneidklinge bzw. die Schneidklingen über den gesamten Einstellbereich stets achsparallel bzw. parallel zur Werkzeugschaftachse ausgerichtet bleiben. Für die Parallelverschiebung der Tragsarme kann beispielsweise eine sog. Parallelogramm-Mechanik eingesetzt werden. Je mehr in Umfangsrichtung verteilte Schneidklingen am Schneidwerkzeug vorgesehen sind, umso kleiner ist die für den Schneidvorgang notwendige Drehbewegung des Schneidwerkzeugs, um einen 360°-Schnitt durchzuführen.

Über die Tragarme kann das Schneidwerkzeug innerhalb der Aorteninnenwandung zentriert werden. Deshalb weist des Schneidwerkzeug vorzugsweise mindestens 3 Tragarme auf.

Die Verwendung von drei Schneidklingen, die auf drei im jeweils 120° Abstand verteilten Tragarmen angeordnet sind, stellt ferner einen guten Kompromiss zwischen platzsparender Verstauung der Tragarme und des zugehörigen Stellmechanismus einerseits und einer möglichst geringen Drehbewegung andererseits.

Gemäß einem Aspekt der Erfindung ist der gesamte distale Abschnitt des Werkzeugschafts, oder zumindest das Schneidwerkzeug samt Schneidklingen vom Werkzeugschaft lösbar. Dadurch kann das Schneidwerkzeug nach dem Schneidvorgang vom Instrument gelöst und anderweitig geborgen werden. Dieser modulare Aufbau ermöglicht des Weiteren einen einfachen Austausch des Schneidwerkzeugs, ohne das gesamte Instrument insgesamt austauschen zu müssen.

Gemäß einem anderen Aspekt der Erfindung wird die Schneideinheit nicht durch ein Schneidwerkzeug mit einer drehbaren Schneidklinge, sondern durch eine zumindest in axialer Richtung relativ zueinander bewegbare Stempel-Matrizen-Anordnung gebildet. Hierbei weist die Stempeleinheit und/oder die Matrizeneinheit eine dünne, biegeelastische, ringförmig gebogene und sich in Umfangsrichtung überlappende Folie auf, deren Kante eine im Wesentlichen ringförmige Schneide bildet. Der Durchmesser dieser ringförmigen Schneide lässt sich somit durch Auf- und Abwickeln der Folie bzw. Relativverschiebung der Folienenden in Umfangsrichtung stufenlos an unterschiedliche Aortendurchmessern anpassen.

Das Grundprinzip dieser als Stanze wirkenden Schneideinheit basiert auf die Verwendung einer dünnen und elastischen Folie, welche zwar in Längsrichtung sehr flexibel ist und zu einem Bogen, Ring und noch weiter gebogen bzw. aufgewickelt werden kann, welche aber in Querrichtung eine relativ scharfe und steife Kante aufweist. Durch ringförmiges Biegen der Folie, des Folienbands oder -streifens und durch entsprechendes Anordnen der Folie um die Schaftlängsachse bildet diese Folie in axialer Richtung, d.h. in Stanz- bzw. Schnittrichtung, eine relativ steife Stanz- bzw. Schneideinheit und erzielt eine Art "Cola-Dosen-Effekt".

Die Folie ist dabei auf der der Matrizeneinheit bzw. der Stempeleinheit zugewandten Seite angeordnet. Durch entsprechende Betätigung der Stempel-Matrizen-Anordnung kann zwischen Stempel und Matrize, wobei zumindest eines von beiden die ringförmige Schneide bzw. Folie aufweist, die Aortenklappe oder ein anderes zu resezierendes Gewebe eingeklemmt und ausgestanzt bzw. durchtrennt werden.

Ein weiterer Vorteil einer solchen weitenverstellbaren Folienklinge ist neben der günstigen Herstellung, die leichte Fixierbarkeit der Folie in der Schneideinheit sowie die selbstständige Weitenverstellung der Folie. Aufgrund der Elastizität der Folie hat diese nach der elastischen Biegeverformung das Bestreben, wieder in ihren Ausgangszustand zurückzukehren. In anderen Worten, die Folie will sich nach dem Aufrollen wieder von selbst abrollen. Diese radiale Vorspannkraft kann zum einen zur Fixierung in einer entsprechenden Halterung in der Schneideinheit ausgenutzt werden (Kraftschluss) und zum anderen kann durch eine geeignete radiale Folienführung dieses Aufrollen derart gesteuert werden, dass die Folie bzw. die Stanzeinheit relativ genau und kreisrund im Durchmesser vergrößert werden kann, ohne dabei aktiv den Durchmesser der zum Ring gebogenen Folie vergrößern zu müssen. Die Folie folgt selbstständig einer Weitenverstellung der Folienführung oder -halterung in Richtung größeren Durchmesser.

Gemäß einem Aspekt der Erfindung kann die Länge der Folie so gewählt werden, dass sie länger als der Umfang des größten einstellbaren Schneiddurchmessers ist. Dadurch wird sichergestellt, dass sich die Folienenden der Folie stets überlappen, wodurch nicht nur eine geschlossene ringförmige Schneide erzielt werden kann, sondern sichergestellt werden kann, dass das äußere Folienende das innere Folienende radial stützt und führt, z.B. bei einer anschließenden Durchmesserverkleinerung und beim Aufwickeln der Folie. Somit ist über gesamten Einstellbereich gewährleistet, dass sich die Folienenden selbst führen.

Gemäß einem Aspekt der Erfindung kann die Stempeleinheit und/oder die Matrizeneinheit eine ringförmige Anordnung mehrerer gebogener, sich in Umfangsrichtung überlappender dünnen biegeelastischen Folien aufweisen, deren Kanten eine im Wesentlichen ringförmige Schneide bilden, deren Durchmesser durch Relativverschiebung der Folien in Umfangsrichtung stufenlos an unterschiedliche Aortendurchmesser anpassbar ist. In diesem Fall wird nicht eine einzelne zu einem Ring gebogene Folie, sondern mehrere hintereinander angeordnete und zum einem Ring gebogenen Folien verwendet, wobei sich benachbarte Folien überlappen. Insbesondere kann es sich hierbei um eine Vielzahl identischer und in Umfangsrichtung gleichmäßig verteilter Folien bzw. Foliensegmente handeln.

Die Verwendung einer Vielzahl an in Umfangsrichtung aneinander gereihter und teilweise überlappender Folien anstelle von einer einzelnen, kreisförmig gebogenen und in Umfangsrichtung überlappenden Folie erhöht den Weiteneinstellbereich. Es hat sich nämlich gezeigt, dass bei der Verwendung einer einzelnen Folie und einem großen Einstellbereich des Schneidendurchmessers die Folie zum Erreichen der kleinen Schneidendurchmesser mehrfach aufgewickelt werden muss. Durch die Mehrfachaufwicklung der Folie erhöht sich nicht nur das Reibmoment der Folien untereinander mit jeder Wicklung (vgl. Seilreibung), sondern es kann auch dazu kommen, dass die Folien an manchen Stellen aufklaffen, was sich wiederum negativ auf die Schneidwirkung der Folie auswirkt. Mehrere kürzere Foliensegmente dagegen zeigen dieses Phänomen nicht und entwickeln beim Reduzieren des Schneiddurchmessers und Zusammenrollen der Foliensegmente untereinander eine geringere Reib- bzw. Klemmkraft als eine mehrfach aufgewickelte einzelne Folie.

Ähnlich wie bereits oben beschrieben sollte die Gesamtlänge aller Folien länger als der Umfang des größten einstellbaren Schneiddurchmessers betragen. Um die gegenseitige radiale Führung der Folien untereinander zu verbessern bzw. überhaupt zu ermöglichen, überlappen sich die Folien in Umfangsrichtung nacheinander abwechselnd außen und innen. Dadurch gleiten die Folien aneinander in- und auseinander.

Vorzugsweise ist die verwendete Folie eine dünne, flexible Metallfolie. Die Metallfolie kann zur Erhöhung der Elastizität auch aus einem superelastischem Material bestehen wie z.B. NiTiNol.

Um die Schneid- bzw. Stanzleistung der Folie weiter zu erhöhen, kann diese bzw. deren Seitenkante(n) mit einem Anschliff versehen sein.

Ferner oder alternativ kann die Folie zur Reduzierung der Stanz- bzw. Schneidkräfte eine Schneidgeometrie aufweisen, bei der nicht die gesamte Schneide auf ein Mal in Eingriff mit dem zu resezierenden Gewebe gelangt. Dies kann insbesondere durch eine gezackte, wellenförmige oder abgeschrägte Schneidkante erreicht werden.

Zur Weitenverstellung der Folienklinge weist die Stempeleinheit und/oder die Matrizeneinheit Folienführungen auf, die über den Stellmechansimus weitenverstellbar sind. Die Folie(n) ist(sind) in den Folienführungen axial und radial so festgelegt, dass sie sich bei Weitenverstellung des Stellmechanismus bzw. bei radialer Bewegung der Folienführungen in Umfangsrichtung verschieben können.

Zum Auf- und Abwickeln der Folie bzw. der Foliensegmente müssen diese die Möglichkeit bekommen, sich in Umfangsrichtung aufzurollen und noch mehr nach innen zu biegen bzw. abzurollen und sich weiter zu entspannen. Durch die axiale Lagerung werden die Stanz- bzw. Schnittkräfte aufgenommen. Die radiale Führung steuert die Durchmessereinstellung der Folienklinge. Durch ein kontrolliertes Verstellen der Folienführungsmimik des Instrumentes (radiale Bewegung) gleitet die Folie bzw. die Foliensegmente durch ihre Vorspannung (Eigenflexibilität) aneinander ab, so dass der Durchmesser der Folienanordnung insgesamt vergrößert bzw. verkleinert werden kann.

Gemäß einem Aspekt der Erfindung fährt der Stempel mit seiner zirkulären bzw. im Wesentlichen ringförmigen Schneide auf die Matrize oder einen Amboss, wie bei einer Knochenstanze.

Alternativ hierzu können sowohl die Stempeleinheit als auch die Matrizeneinheit eine erfindungsgemäße Folienklinge aufweisen. In diesem Fall können deren Folienführungen bzw. Führungsmimiken so ausgelegt sein, dass sich die Schneiden von Stempeleinheit und Matrizeneinheit in ihren Schneidurchmessern jeweils so unterscheiden, dass sich die beiden Schneiden aneinander vorbeibewegen bzw. scheren bzw. in axialer Richtung überlappen. Dadurch wird die Schnittleistung verbessert. Es ist auch denkbar, dass die beiden Schneiden direkt aufeinandertreffen.

Um die Handhabung des erfindungsgemäßen Schneidinstruments für den Operateur zu erleichtern und um sicherzustellen, dass Stempel und Matrize bzw. die zusammenwirkenden Schneiden stets die richtige Weite bzw. richtige Durchmesserdifferenz aufweisen, können die weitenverstellbare Führungsmimik der Stempeleinheit und die weitenverstellbare Führungsmimik der Matrizeneinheit miteinander gekoppelt sein. Die synchrone und analoge Weitenverstellung beider Einheiten kann insbesondere über eine entsprechende Verstellmechanik und einem am proximalen Ende des Werkzeugschafts angeordneten Handstück oder motorische Einrichtung erfolgen.

Gemäß einem Aspekt der Erfindung sind die Tragarme über eine oder mehrere Federn radial nach außen federvorgespannt. Diese Federn dienen als Energiespeicher für den Aufspannmechanismus des Schneidwerkzeugs. Wenn die Tragarme freigegeben werden, spannen sich die Tragarme über die Federvorspannung von alleine auf, bis sie in Anlage mit der Aorteninnenwandung kommen, die eine Art Anschlag des selbsttätigen Aufspannmechanismus bilden. Über die Federvorspannung lässt sich auch die Radialkraft der Tragarme voreinstellen. Über einen Rückholmechanismus könnene die Tragarme wieder eingeholt werden.

Damit die künstliche Herzklappe möglichst nicht migrieren kann, ist es von Vorteil, wenn die natürliche Klappe nicht ganz genau an der Aorteninnenwandung abgetrennt wird, sondern wenn ein gleichbleibender Ansatz stehen bleibt, der die künstliche Klappe in axialer Richtung fixiert. Deshalb kann die Folienführung mit einem Abstandhalter versehen sein bzw. kann die Folie in der Führungsmimik um ein bestimmtes Maß radial innerhalb der Außenabmessung der Führungsmimik geführt sein, so dass sichergestellt wird, dass die Aortenwand beim Stanzen nicht in Mitleidenschaft gezogen wird. Gemäß einem Aspekt der Erfindung kann lediglich der Außendurchmesser der Führungsmimik um einen vorbestimmten Abstand größer als der Durchmesser der ringförmigen Schneide sein. Über die radial nach außen gestellten bzw. aufgespreizten Folienführungen kann die Aorteninnenwand aufgespannt werden, während die radial weiter innen liegenden Schneiden die Aortenklappe abtrennen.

Da die Außenabmessungen der Schneideinheit im Wesentlichen durch die Führungsmimik bedingt ist, ist es vorteilhaft, wenn die Führungsmimik im Werkzeugschaft versenkbar ist und die durch die zumindest eine Folie gebildete ringförmige Schneide im Wesentlichen auf den Durchmesser des Werkzeugschafts reduzierbar ist. Dies ermöglicht das Durchführen der Schneideinheit durch die Aortenklappe, ohne dabei Kalkablagerungen abzukratzen und abzulösen.

Gemäß einem Aspekt der Erfindung kann die Führungsmimik durch mehrere in Umfangsrichtung verteilte, radial verstellbare und mit einander gekoppelte Führungsarme gebildet sein, die über eine Parallelogramm-Mechanik parallel zur Werkzeugschaftachse verschiebbar sind, so dass die Folien stets achsparallel bzw. parallel zur Stanzachse ausgerichtet und geführt sind.

Die Führungsarme sind jeweils über zwei Gelenkstangen an zwei axial relativ zueinander bewegbaren Werkzeugschaftkomponenten angelenkt. Durch axiale Verschiebung der beiden Werkzeugschaftkomponenten werden die Führungsarme über die Gelenkstangen radial nach außen gestellt bzw. radial nach innen gezogen. Diese Umwandlung einer axialen Bewegung eines Werkzeugschaftantriebs in eine radiale Bewegung der Führungsarme ist nicht nur einfach handhab- und einstellbar, sondern auch sehr platzsparend.

Gemäß einem Aspekt der Erfindung entspricht die Anzahl der Folien der Anzahl der Führungsarme. Die Folienlängen sind dabei größer als der Kreisbogenabschnitt zwischen zwei Führungsarmen beim größten einstellbaren Schneiddurchmesser.

Gemäß einem Aspekt weist die Führungsmimik zumindest vier in Umfangsrichtung gleichmäßig verteilte und radial verstellbare Führungsarme auf. Dadurch werden die radial nach außen strebenden Folien zumindest an vier Punkten radial gehalten.

Ferner kann jede Folie mit einem Folienende an einem entsprechenden Führungsarm fest, vorzugsweise stoffschlüssig verbunden sein. Dadurch wird sichergestellt, dass die Folien ihre Umfangsposition bzw. ihre Relativposition gegenüber benachbarter Folien beibehält. Zum anderen wird erreicht, dass die Folie am Werkzeug befestigt ist und nicht aus der Führung herausfallen kann, ohne dabei jedoch die Biegung der Folie und das Gleiten der Folien in- und auseinander zu behindern.

Um den Gleitwiderstand zwischen den Folien bzw. deren Überlappungsabschnitten zu reduzieren, um so das Auf- und Abwickeln der Folien zu erleichtern, können zwischen den Folien Gleitblättchen oder eine Gleitlage zwischengeschaltet werden. Alternativ oder zusätzlich können die entsprechenden aneinander abgleitenden Folienoberflächen oder zumindest Abschnitte hiervon mit einer Gleitbeschichtung versehen werden.

Gemäß einem Aspekt der Erfindung können die Folien in der weitenverstellbaren Folienführung in radialer Richtung lediglich außen abgestützt sein. Die gebogenen Folien folgen nämlich aufgrund ihrer Eigenflexibilität selbstständig einer Weitenverstellung der Führungsarme radial nach außen. Dies ist insbesondere dann von Vorteil, wenn das Instrument bei der Operation nur in eine Richtung verstellt werden soll, d.h. wenn die Schneideinheit nur von einer komprimierten Ausgangsstellung auf den zum Aortendurchmesser passenden Durchmesser vergrößert werden soll. In diesem Fall folgt die zunächst aufgerollte Folie ohne weiteres Zutun von sich der Aufweitung der Folienführung.

Alternativ können die Folien in Axialnuten oder -schlitzen an Stirnseiten der Führungsarme so aufgenommen sein, dass sie aus diesen zumindest um die zu erwartenden Schnitttiefe in axialer Richtung vorstehen. In diesen Axialnuten sind diese Folien axial am Nutgrund und radial innen und radial außen an den Nutflanken abgestützt. Diese Nuten sind in radialer Richtung so breit bemessen, dass sie in der komprimierten Ausgangsstellung mehrere Folienlagen aufnehmen können. Im aufgespannten Zustand ergibt sich an sich ein größeres radiales Spiel der Folien innerhalb der Nuten. Dieser Spalt spielt grundsätzlich jedoch keine große Rolle, da sich die Folien von alleine nach außen drücken und den Spalt ausgleichen.

Um jedoch sicherzustellen, dass die Folien bei axialer Belastung nicht doch verkippen oder radial ausweichen, kann eine Spaltausgleichseinrichtung vorgesehen sein, welche den Abstand zwischen den Folienlagen und den Nutwandungen je nach Abwicklungszustand bzw. Weiteneinstellung anpasst bzw. minimiert. Die Spaltausgleichseinrichtung kann auch in Form einer Klemm- oder Spanneinrichtung ausgebildet sein, welche die Folie vor dem Schneidvorgang in den Axialnuten in radialer Richtung verspannt oder einklemmt.

Die Spaltausgleichseinrichtung bzw. die Klemm- oder Spanneinrichtung kann gemäß einem Aspekt der Erfindung ein in der Axialnut angeordnetes federelastisches Element sein, welches die Folie bzw. die Folienlagen gegen eine der Nutinnenwandungen, vorzugsweise gegen die radial äußere Nutinnenwandung drückt. Die Druckkraft des federelastischen Elements kann so bemessen sein, dass sie einerseits ein Aneinanderabgleiten der Folienlagen zum Auf- und Abrollen der Folien in Umfangsrichtung zulässt, ein radiales Ausweichen oder Verkippen der Folien innerhalb der Nut jedoch verhindert.

Anstelle eines federelastischen Elements innerhalb der Nut kann die Klemm- oder Spanneinrichtung so ausgeführt sein, dass zumindest eine der Nutwandungen radial nachführbar ist und den sich beim Abwickeln der Folien ergebenden Spalt ausgleichen kann. In einer bevorzugten Ausführungsform wird die radial innere Nutwandung radial nach Außen nachgeführt. Beim Aufwickeln gibt diese entsprechend nach Innen nach. Das Nachführen der Nutwandung kann ebenfalls durch ein Federelement realisiert werden.

Die Klemm- oder Spanneinrichtung kann auch durch ein Bremsbackensystem realisiert werden, welches die Folien zur Weiteneinstellung freigibt und unmittelbar vor dem Schneidvorgang, z.B. mittels des Handstücks, aktivierbar ist.

Um zu gewährleisten, dass sich die (Metall-)Folien immer eng aneinanderlegen, damit die Folie in der Schneideinheit und die Folie in der Matrizeneinheit beim Stanz- bzw. Schneidvorgang ineinander eindringen können, ohne auf der andere Folie aufzusetzen, kann durch eine entsprechende Formgebung der Folienenden das Widerstandsmoment gesenkt werden. Hierzu kann zumindest ein Folienende abgeschrägt bzw. spitz oder rund zulaufend sein.

Um die Schnittleistung weiter zu verbessern, kann die Stempeleinheit und/oder die Matrizeneinheit beim Stanzvorgang eine Schraubbewegung oder ein vorbestimmte überlagerte axiale und rotatorische Bewegung durchführen. Dies kann beispielweise durch entsprechende Kulissen im Werkzeugschaft erreicht werden, welche die Stempeleinheit und/oder Matrizeneinheit, wenn sie sich beim Schneidvorgang axial aufeinander zu bewegen, zu einer Drehbewegung zwingen.

Über die Folienführungen bzw. Tragarme kann gemäß einem Aspekt der Erfindung ein engmaschiges, elastisches Netz aufgesetzt sein, welches der Verstellung des Schneidendurchmessers folgt und beim Stanz- bzw. Schneidvorgang einen geschlossenen Raum bildet, in dem die abgetrennten Aortenklappen aufgefangen und sicher geborgen werden können.

Je nach Anwendungsfall kann es von Vorteil oder notwendig sein, wenn der distale Teil der Stempel-Matrizen-Anordnung bzw. der hinter der Aortenklappe befindliche Abschnitt des erfindungsgemäßen Werkzeugschafts nach der Aortenklappenresektion nicht wieder zusammen mit dem Instrument herausgeführt wird, sondern vom Instrument gelöst und anderweitig geborgen werden kann. Die beiden Schneidwerkzeuge können auch vor der Aortenresektion getrennt voneinander eingeführt werden und im sog. Rendezvous-Verfahren intrakorporal miteinander verbunden werden. Deshalb können gemäß einem Aspekt der Erfindung die Stempeleinheit und die Matrizeneinheit lösbar miteinander gekoppelt sein, so dass der distale Teil von beiden vor oder nach der Aortenklappenresektion vom Werkzeugschaft gekoppelt bzw. entkoppelt werden kann.

Gemäß einem Aspekt der Erfindung kann der Instrumentenschaft flexibel ausgeführt sein. In diesem Fall weist der Instrumentenschaft eine zentrale axiale Durchgangsbohrung zur Aufnahme eines Führungsdrahts. Der Führungsdraht kann an seinem distalen Ende eine aufweitbare Positioniervorrichtung wie z.B. einen Spreizmechanismus, einen Ballon oder Ähnliches aufweisen, um den Führungsdraht in der Nähe der Aortenklappe zu positionieren und zu fixieren. Der flexible Instrumentenschaft kann anschließend über den Führungsdraht zur Aortenklappe geführt werden.

Die zuvor genannten Merkmale werden einzeln und in Kombination beansprucht.

Die vorliegende Erfindung wird anhand von beigefügten Zeichnungen näher dargestellt.

Es zeigen:
- Fig. 1: ein erfindungsgemäßes Schneidinstrument gemäß einer ersten Ausführungsform der Erfindung;
- Fig. 2: eine perspektivische Ansicht des Instrumentenschafts mit einer zusammengeklappten Schneideinheit (ohne Folienklinge) gemäß der ersten Ausführungsform;
- Fig. 3: eine perspektivische Ansicht des Instrumentenschafts mit einer aufgespreizten Schneideinheit (ohne Folienklinge) gemäß der ersten Ausführungsform;
- Fig. 4: eine Querschnittsansicht des Instrumentenschafts mit einer zusammengeklappten Schneideinheit (ohne Folienklinge) gemäß der ersten Ausführungsform;
- Fig. 5: eine Querschnittsansicht des Instrumentenschafts mit einer aufgespreizten Schneideinheit (mit Folienklinge) gemäß der ersten Ausführungsform;
- Fig. 6: eine vergrößerte Querschnittsdetailansicht von Folienführungen der Schneideinheit gemäß der ersten Ausführungsform;
- Fig. 7: eine vergrößerte Querschnittsdetailansicht der Aorta nach einer Aortenklappenresektion;
- Fig. 8: eine ausgewickelte einer Folienklinge im Ausgangszustand;
- Fig. 9A bis 9D: verschiedene Kantengeometrien der Folienklinge;
- Fig. 10: eine schematische Ansicht einer durch mehrere Folien gebildete Folienklinge gemäß einer zweiten Ausführungsform mit einem mittleren Schneiddurchmesser;
- Fig. 11: eine schematische Ansicht der durch mehrere Folien gebildete Folienklinge gemäß der zweiten Ausführungsform mit einem maximalen Schneiddurchmesser;
- Fig. 12: eine schematische Ansicht der durch mehrere Folien gebildete Folienklinge gemäß der zweiten Ausführungsform mit einem minimalen Schneiddurchmesser;
- Fig. 13: eine perspektivische Teilansicht eines Schneidinstruments gemäß einer dritten Ausführungsform der Erfindung; und

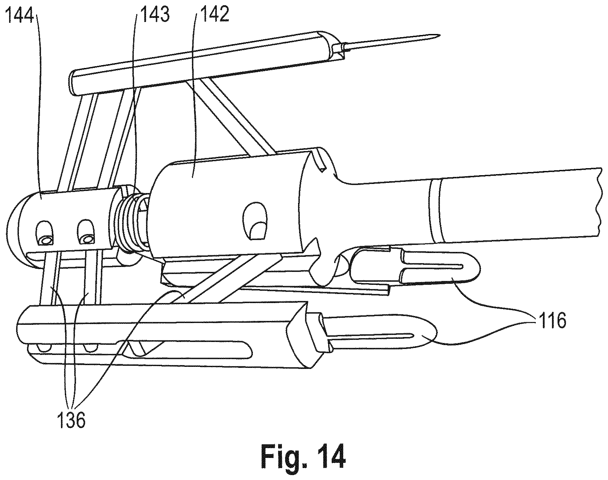

- Fig. 14: eine vergrößerte Ansicht der Schneideinheit des Schneidinstruments gemäß der dritten Ausführungsform der Erfindung.

### Detaillierte Beschreibung von Ausführungsformen

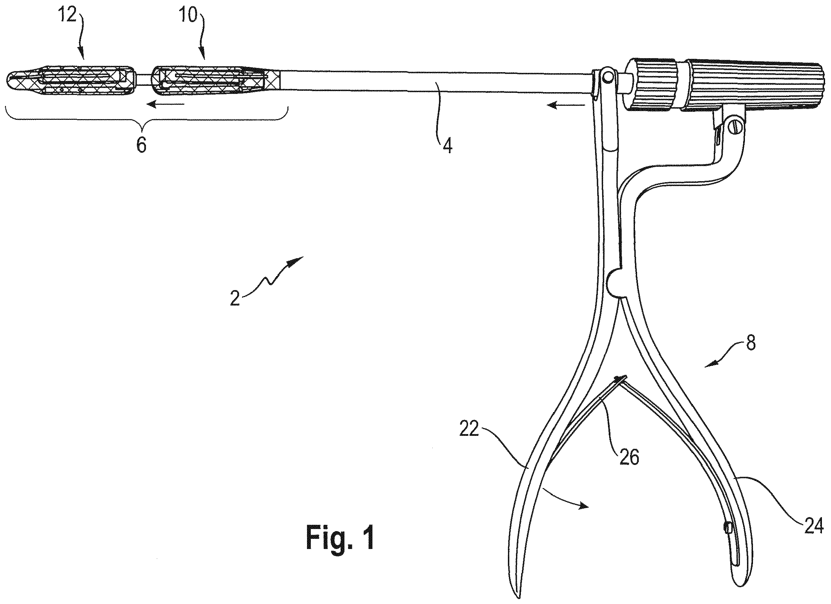

Die Figur 1 zeigt ein erfindungsgemäßes Schneidinstrument 2, das am distalen Ende eines Werkzeugschafts 4 eine Schneideinheit 6 aufweist, die über ein am proximalen Ende des Werkzeugschafts 4 angeordnetes Handstück 8 betätigbar ist. Der Schaft 4 kann starr oder flexibel, ggf. über das Handstück 8 manipulierbar sein. Die Schneideinheit 6 ist im Wesentlichen als Stempel-Matrizen-Anordnung ausgebildet und besteht aus zwei axial am Werkzeugschaft 4 angeordnete und über das Handstück 8 relativ zu- und auseinander bewegbaren Schneidwerkzeugen 10 und 12. Genauer gesagt fungiert das proximale Schneidwerkzeug 10 im dargestellten Beispiel als bewegbare Stempeleinheit und das distale Schneidwerkzeug 12 als statische Matrizeneinheit. Selbstverständlich kann in einer alternativen Ausgestaltung auch das distale Schneidwerkzeug 12 zum proximalen Schneidwerkzeug 10 oder beide aufeinander zu bewegbar sein.

Die Schneidwerkzeuge 10, 12 weisen jeweils eine Schneide 14 auf, deren Aufbau und Funktionsweise weiter unten detailliert beschrieben wird.

Das Handstück 8 weist zwei ergonomisch geformte, gelenkig verbundene Handgriffe 22 und 24 auf, die über eine Feder 26 in eine Ausgangslage vorgespannt sind. In der Ausgangslage sind die Schneidwerkzeuge 10, 12 zueinander beabstandet.

Der Werkzeugschaft 4 ist mehrteilig aufgebaut und weist eine erste Schafteinheit 28 auf, die zum einen mit dem Stempel 10 und zum anderen gelenkig mit einem der Handgriff 22, 24, nämlich Handgriff 22 verbunden ist. Die Schafteinheit 28 ist in axialer Richtung relativ zu einer zweiten Schafteinheit 30 bewegbar, die zum einen mit der Matrize 10 und zum anderen gelenkig mit dem anderen der beiden Handgriffe 22, 24, nämlich Handgriff 24, verbunden ist. Der Benutzer des Instruments 2 kann die Handgriffe 22 und 24 mit seinen Fingern und Handballen greifen und entgegen der Federkraft der Feder 26 betätigen, so dass der Stempel 10 axial auf die Matrize 12 zu bewegt wird und ggf. dazwischen liegendes Gewebe durchstanzt bzw. durchtrennt.

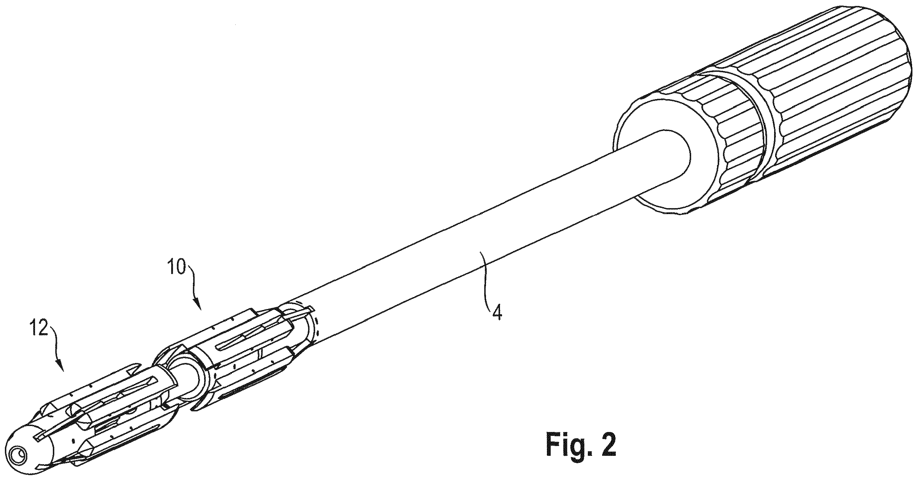

Sowohl das als Stempel wirkende Schneidwerkzeug 10 als auch das als Matrize wirkende Schneidwerkzeug 12 sind über eine Stellmechanismus weitenverstellbar, dessen Aufbau und Funktionsweise im Folgenden detailliert dargestellt wird, wobei die Fig. 2 bis Fig. 5 in einer vereinfachten Darstellung lediglich den Werkzeugschaft 4 ohne Handstück 8 zeigen.

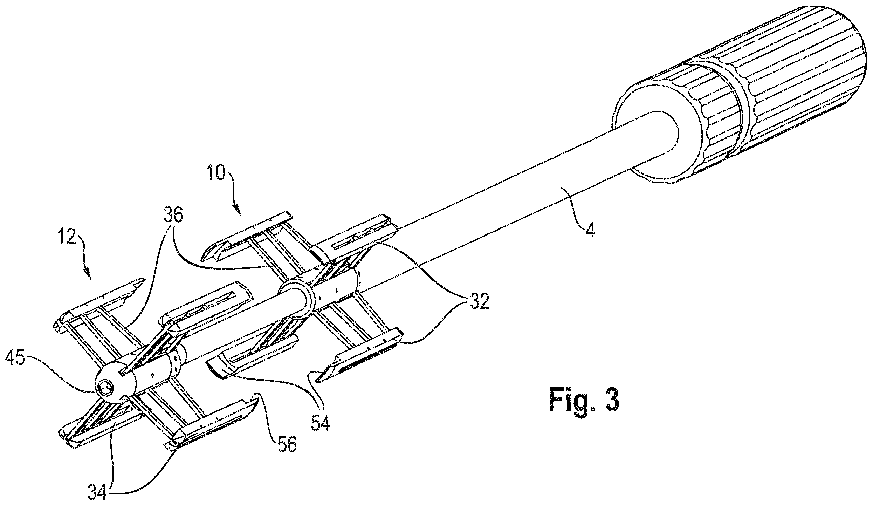

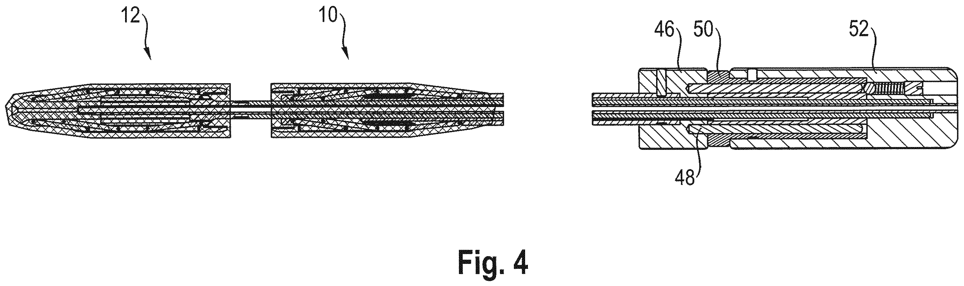

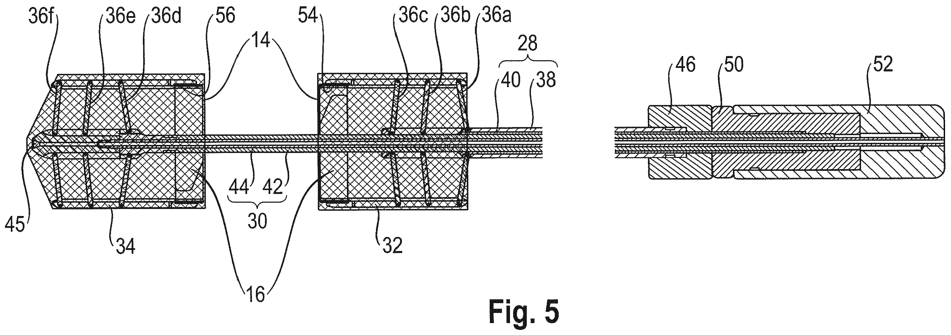

Die Schneideinheit 6 bzw. die Schneidwerkzeuge 10 und 12 lassen sich zwischen einer komprimierten oder zusammengelegten Stellung, in der die Schneidwerkzeuge 10 und 12 ihren minimalen Durchmesser aufweisen (siehe Fig. 2 und Fig. 4), und einer aufgespreizten Stellung, in der die Schneidwerkzeuge 10 und 12 ihren maximalen Durchmesser einnehmen (siehe Fig. 3 und Fig. 5), stufenlos einstellen.

Jedes Schneidwerkzeug 10 und 12 weist mehrere (im vorliegenden Beispiel vier) in Umfangsrichtung gleichmäßig verteilte und sich in axialer Richtung bzw. parallel zum Werkzeugschaft 4 erstreckende Führungen oder Führungsarme 32 bzw. 34 auf. Diese Führungsarme 32, 34 sind jeweils über mehrere Gelenkarme 36 am Werkzeugschaft 4 geführt und lassen sich über diese Gelenkarme 36 radial zum Werkzeugschaft 4 verstellen, d.h. nach außen aufspreizen bzw. nach innen zusammenlegen.

Die erste Schafteinheit 28 (siehe Fig. 5) des ersten Schneidwerkzeugs 10 besteht aus einem äußeren Hohlschaft 38 und einem innerhalb dessen axial bewegbaren zweiten inneren Hohlschaft 40. Von den Gelenkarme 36 des entsprechenden Führungsarms 32 ist ein Gelenkarm 36a gelenkig mit dem äußeren Hohlschaft 38 verbunden, während zwei andere Gelenkarm 36b, 36c gelenkig mit dem inneren Hohlschaft 40 verbunden sind. Die Gelenke, über welche die Gelenkarme 36 mit den Führungsarmen 32 und mit dem äußeren Hohlschaft 38 bzw. inneren Hohlschaft 40 verbunden sind, definieren ein Parallelogramm. Diese Parallelogramm-Mechanik stellt sicher, dass die Führungsarme 32 bei der Weitenverstellung stets parallel zum Werkzeugsschaft 4 ausgerichtet bleiben, wenn die beiden Hohlschäfte 38, 40 auseinander bzw. ineinander bewegt werden und dabei über die Gelenkarme 36 daran angelenkten Führungsarme radial nach außen drücken bzw. nach innen ziehen (vgl. hierzu Fig. 2 und Fig. 3). Durch die drei Gelenkarme 36a, 36b, 36c wird die Lage bzw. die Ausrichtung der Führungsarme 32 eindeutig definiert, so dass die Führungsarme 32 bei einer Relativverschiebung der Schäfte 38, 40 zueinander nicht verkippt.

Die zweite Schafteinheit 30 des zweiten Schneidwerkzeugs 12 ist analog zur ersten Schafteeinheit 28 aufgebaut und weist ebenfalls zwei zueinander axial bewegbare Schaftteile, nämlich einen äußeren Hohlschaft 42 und einen darin axial geführten und bewegbaren Schaft 44. Zu beachten ist, dass die zweite Schafteinheit 30 innerhalb der ersten Schafteinheit 28 aufgenommen und darin axial bewegbar ist. Auch die zweite Schafteinheit 30 weist die zuvor detailliert beschriebene Parallelogramm-Mechanik auf, mit der über axial relativ zueinander verschiebbare Schaftteile 42, 44 die Führungsarme 34 parallel zu den Schaftteilen 42, 44 bzw. zum Werkzeugschaft 4 verschoben werden. Hierbei ist ein Gelenkarm 36d mit dem äußeren Hohlschaft 42 und zwei Gelenkarme 36e, 36f mit dem inneren Schaft 44 verbunden.

Am distalen Ende des Instrumentenschafts 4 ist eine zentrale Axialbohrung 45 (siehe Fig. 3 und 5) für einen Führungsdraht vorgesehen, die sich durch den gesamten Instrumentenschaft 4 hindurch erstreckt. Über einen zuvor in der Nähe der Aortenklappe positionierten und durch den Instrumentenschaft geführten Führungsdraht kann die Schneideinheit 6 zur Aortenklappe geführt werden.

Im vorliegenden Beispiel ist die Weitenverstellung der beiden Schneidwerkzeuge 10 und 12 mechanisch miteinander gekoppelt, so dass deren Durchmesser gemeinsam und analog radial vergrößert bzw. verkleinert werden.

Die Relativbewegung der beiden Schaftteile 38, 40 der ersten Schafteinheit 28 und der beiden Schaftteile 42, 44 der zweiten Schafteinheit 30 erfolgt über einen Gewindetrieb, der über eine an einem am proximalen Ende des Werkzeugschaft 4 angeordneten Drehknopf 46 betätigt wird. Eine dort eingegebene Drehbewegung wird in eine translatorische Relativverschiebung der jeweiligen Schaftteile 38, 40, 42, 44 zueinander umwandelt. Diese zur Weitenverstellung notwendige Mechanik ist freigegeben, wenn sich die die Handgriffe 22, 24 in ihrer Ausgangslage befinden, und blockiert, wenn die Handgriffe 22, 24 zum Schneiden betätigt werden.

Fig. 4 zeigt eine Querschnittsansicht des Werkzeugschafts 4 ohne Handstück 8. Der Drehknopf 46 ist mit dem äußeren Hohlschaft 38 der ersten Schafteinheit 28 axial gekoppelt und zu diesem relativ drehbar. Der Drehkopf 46 steht ferner im Gewindeeingriff mit dem innerem Hohlschaft 40 der ersten Schafteinheit 28, so dass eine Drehbewegung des Drehknopf 46 zu einer axialen Relativverschiebung der beiden Hohlschäfte 38, 40 führt, ohne diese jedoch zueinander zu verdrehen.

Der Drehknopf 46 ist über einen exzentrisch angeordnete Axialstift 48 mit einem Drehteil 50 drehfest koppelt, welches wiederum mit einem Schaftendstück 52 axial gekoppelt und zu diesem relativ drehbar ist. Dieses Schaftendstück 52 ist mit dem inneren Schaft 44 der zweiten Schafteinheit 30 fest verbunden, während sich das Drehteil 50 im Gewindeeingriff mit dem äußeren Hohlschaft 42 der zweiten Schafteinheit 30 befindet. Somit führt eine Drehung des Drehknopfs 46 nicht nur zur Relativverschiebung der Schafteile 38, 40 der ersten Schafteinheit 28 und der damit verbundenen Weitenverstellung des ersten Schneidwerkzeugs 10, sondern über die Kopplung mit dem Drehteil 50 auch zu einer Relativverschiebung der Schafteile 42, 44 der zweiten Schafteinheit 30 und einer damit verbundenen Weitenverstellung des zweiten Schneidwerkzeugs 12. Durch diese Kopplung werden die Schafteinheiten 28, 30 bzw. Schneidwerkzeuge 10, 12 synchron in ihrer Weite eingestellt.

Im Folgenden wird auf den Aufbau und die Weitenverstellung der Ringschneide 14 eingegangen.

Die Ringschneide 14 wird nicht durch ein statisches ringförmiges Schneidelement, sondern durch ein dünnes und schmales flexibles Folienband 16 gebildet, das aus Metall oder einer Metalllegierung, insbesondere aus NiTiNol, oder aus einem anderen geeigneten biegeelastischen Material bestehen kann. Die Folie 16 oder zumindest deren Kante 18 sollte so dünn sein, dass sich die Kante 18 bei einem gewissen Druck und ggf. zusätzlichen Drehbewegung eine Schneidwirkung für menschliches Gewebe erzielt. Zur Schärfung der Kante 18 kann diese zusätzlich mit einem Anschliff versehen sein.

Während das längliche Folienband 16 in Längsrichtung elastisch biegsam ist, ist es in Querrichtung relativ steif. Wenn die Folie 16 zu einem Ring gebogen, so dass sich die Folienenden überlappen, und - wie in der Fig. 5 dargestellt - in entsprechende Aufnahmen 54 bzw. 56 der beiden Schneidwerkzeuge 10, 12 eingesetzt ist, erhöht sich die Steifigkeit der Folie 16 so, dass sie als Schneide verwendet werden kann. Aufgrund ihrer biegeelastischen Vorspannung schmiegen sich die Überlappungsabschnitte 20 der Folie 16 dicht aneinander, so dass die Kante 18 der Folie 16 eine im Wesentlichen geschlossene und kreisförmige Ringschneide 14 bilden.

Die Aufnahmen 54 bzw. 56 befinden sich an einander gegenüberliegenden axialen Enden der Führungsarme 32 und 34. Die Breite der Folien 16 ist so bemessen, dass die Folien 16 aus den Aufnahmen 54, 56 etwas axial vorstehen und zwar um die zu erwartende Schnitttiefe.

Diese Aufnahmen 54, 56 sind im Wesentlichen radiale Absätze in den Führungsarmen 32, 34, welche die Folie 16 lediglich in einer axialen Richtung und nur radial außen stützen. Auf Grund der Eigenelastizität der Metallfolie 16 hat diese nach der Verformung das Bestreben, wieder in ihren Ausgangszustand zurückzukehren. Durch diese Rückstellkraft wird die Folie 16 in den Aufnahmen 54, 56 der vier in Umfangsrichtung gleichmäßig verteilten Führungsarmen 32, 34 kraftschlüssig eingespannt.

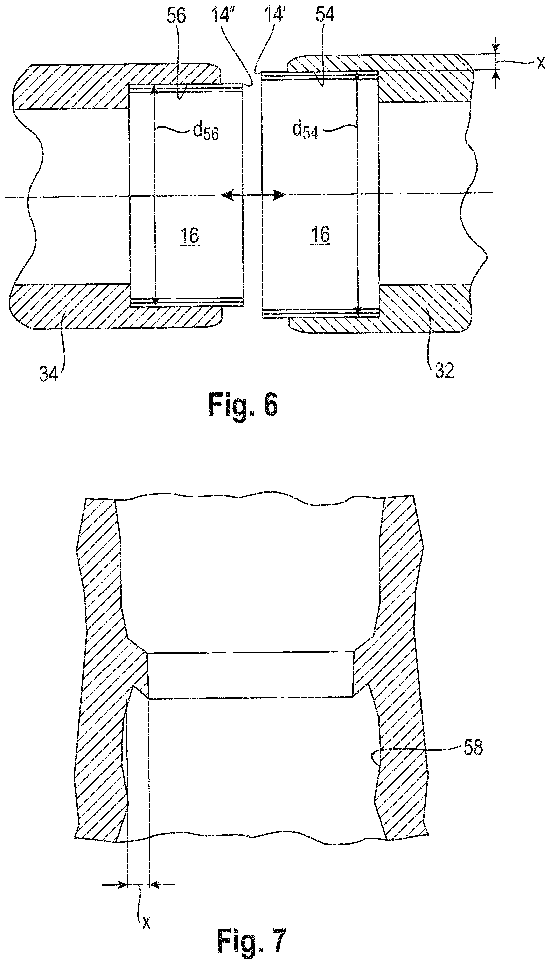

Die Aufnahmen bzw. Absätze 54, 56 in der Führungsarme 32, 34 von Stempel 10 und Matrize 12 können sich unterscheiden und, wie in der Querschnittsansicht in der Fig. 6 dargestellt, eine unterschiedliche radiale Tiefe bzw. einen unterschiedlichen Durchmesser d₅₄, d₅₆ aufweisen. Dieser Durchmesserunterschied im radialen Auflagebereich der Metallfolien 16 führt zu einem Durchmesserunterschied in den von den Folien 16 gebildeten Ringschneiden 14. Dieser Durchmesserunterschied wird so gewählt, dass die Ringschneide 14 des Stempels 10 und die Ringschneide 14 der Matrize scheren bzw. sich aneinander vorbeibewegen lassen.

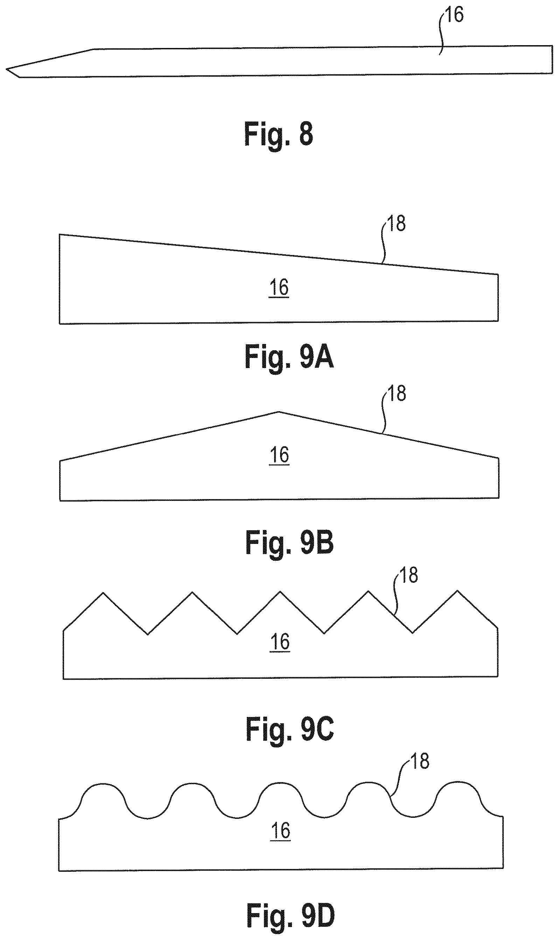

Um zu gewährleisten, dass sich die Metallfoliensegmente 16 immer eng aneinanderschmiegen und die Ringschneide 14 des Stempels 10 beim Stanzvorgang in die Matrize 12 bzw. in deren Ringschneide 14 eindringen kann, ohne dass diese kollidieren, wird durch eine entsprechende Form der Folienenden das Widerstandsmoment gesenkt. Hierfür sind die Endabschnitte der Folien, insbesondere in Einführrichtung, abgeschrägt und ggf. abgerundet (siehe Fig. 8).

Ferner befinden sich die Aufnahmen 54, 56 nicht unmittelbar im äußeren Bereich der Führungsarme 32, 34, sondern sind um einen gewissen Abstand x radial weiter innen angeordnet, so dass beim Schneidvorgang zum einen die Aortenwandung 58 beim Stanzen nicht in Mitleidenschaft gezogen wird und zum anderen die natürliche Aortenklappe nicht ganz genau an der Aorteninnenwandung 58 abgetrennt wird, sondern ein gleichbleibender Ansatz x für die axiale Fixierung der künstlichen Klappe verbleibt (siehe Fig. 7). Die äußeren Abschnitte der Führungsarme 32, 34 dienen sozusagen als Abstandshalter zum Abstützen bzw. Aufspannen der Aorteninnenwandung 58.

Da wie oben bereits dargestellt die Führungsarme 32, 34 über eine Parallelogramm-Mechanik radial nach außen parallel verschoben werden, sind die Aufnahmen 54, 56 und die darin aufgenommen Folien 16 stets parallel zur Stanzachse ausgerichtet und geführt sind, um so mit ihren Kanten 18 ihre maximal Schnittwirkung zu verwirklichen.

Der Durchmesser der durch die Folie 16 gebildeten Ringschneide 14 kann durch radiales Verstellen der Führungsarme 32, 34 stufenlos eingestellt werden, wobei dabei die überlappenden Folien 16 aneinander abgleiten.

Wenn beispielsweise die Weite der Führungsarme 32, 34 vergrößert wird, wandern die Aufnahmen 54, 56 radial nach außen und die darin eingespannten Folien 16 folgen den Aufnahmen 54, 56 selbstständig aufgrund ihrer biegeelastischen Vorspannung. Untereinander gleiten die Folien 16 an ihren Überlappungsabschnitten 20 ab. Die maximale Weitenverstellung wird durch die Länge der Folie 16 begrenzt und ist nur soweit möglich, solange sich die Folienenden noch überlappen.

Wenn dagegen die Weite der Führungsarme 32, 34 verringert wird, wandern die Aufnahmen 54, 56 radial nach innen und die darin eingespannten Folien 16 werden weiter nach innen gedrückt, wodurch die Folienenden aneinander gleiten und sich die Folie 16 weiter aufrollt.

Für die Weitenverstellung der Folien 16 ist es notwendig, dass diese in Umfangsrichtung aus- und ineinander gleiten können und so einen bestimmten Freiheitsgrad in Umfangsrichtung benötigen. Um jedoch ein Herausrutschen oder -fallen der Folien 16 aus den Führungsarmen 32, 34 in axialer Richtung zu verhindern oder um zu verhindern, dass die Folien 16 in Umfangsrichtung immer gleich beanstandet bleiben, kann jeweils ein Ende oder ein mittlerer Abschnitt der Folienbänder 16 an dem entsprechenden Führungsarm 32, 34 angeklebt, angelötet oder anderweitig befestigt sein, während das andere Ende frei beweglich bleibt. Vorzugsweise wird bei der Verwendung einer Folie ein Folienende und bei der Verwendung von mehreren Foliensegmenten jeweils ein mittlerer Abschnitt des Foliensegments fixiert.

Um zu verhindern, dass die gesamte Schneide 14 gleichzeitig mit dem Gewebe in Kontakt kommt, kann die Folie 16 unterschiedliche Schneidengeometrien mit z.B einer schrägen, gezackten oder wellenförmigen Schneidkante 18 aufweisen (siehe Fig. 9A bis 9B), um so die Schneid- bzw. Stanzkräfte zu reduzieren.

Im Folgenden wird eine zweite Ausführungsform der Erfindung beschrieben, bei welcher ein identisches Schneidinstrument verwendet wird, jedoch als Folienklinge nicht eine einzelne Folie, sondern mehrere in Umfangsrichtung angeordnete Foliensegmente 16 verwendet werden.

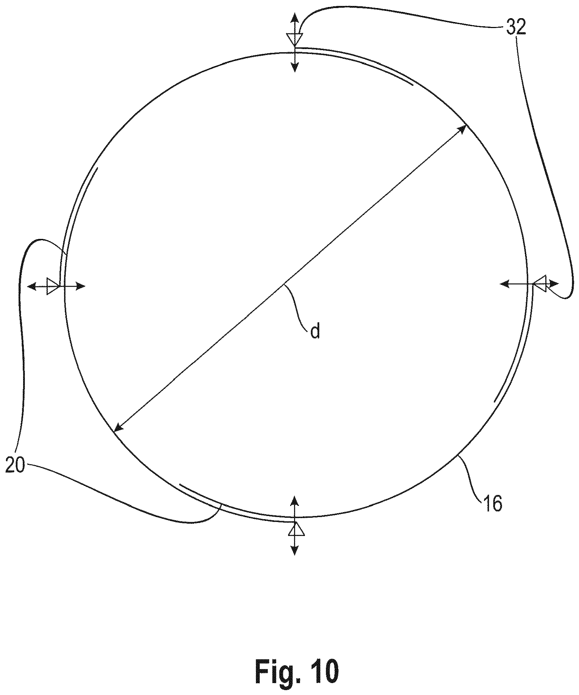

Wie in der Fig. 10 schematisch und exemplarisch für ein Schneidwerkzeug (Stempel oder Matrize) dargestellt, werden mehrere (im Vergleich zur ersten Ausführungsform kürzere) Folien 16 in deren Längserstreckung so nacheinander angeordnet, dass sie einander abwechselnd außen und innen überlappen. Diese Folienanordnung wird anschließend zu einem Kreisring gebogen, so dass sich die erste und die letzte Folie 16 ebenfalls überlappen, und anschließend - wie bei der ersten Ausführungsform - in entsprechende Aufnahmen 54 bzw. 56 eingesetzt, die an einem axialen Ende der Führungsarme 32 bzw. 34 ausgebildet sind. Aufgrund ihrer biegeelastischen Vorspannung schmiegen sich die Überlappungsabschnitte 20 der Folien dicht aneinander, so dass die Kanten 18 der Anordnung der vier Folien 16 eine im Wesentlichen geschlossene und kreisförmige Ringschneide 14 bilden.

Auf Grund der Eigenelastizität der Metallfoliensegmente 16 haben diese ebenfalls nach der Verformung das Bestreben, wieder in ihren Ausgangszustand zurückzukehren. Durch diese Rückstellkraft werden die Folien 16 in den Aufnahmen 54, 56 der vier in Umfangsrichtung gleichmäßig verteilten Führungsarmen 32, 34 kraftschlüssig eingespannt.

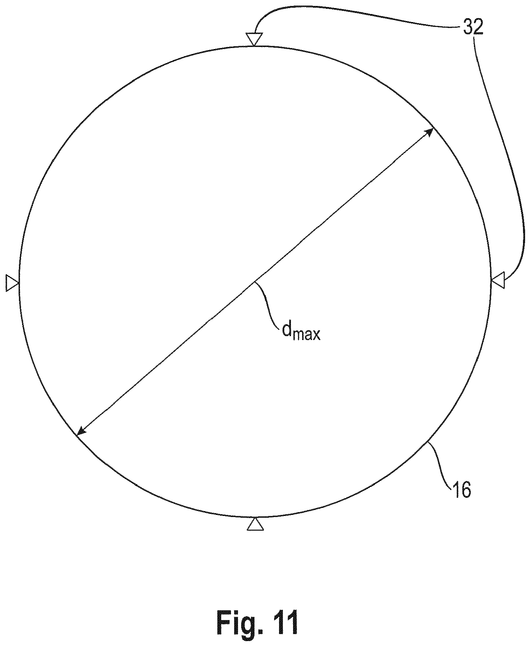

Wie oben detailliert beschrieben kann der Durchmesser der durch die Folienanordnung gebildete Ringschneide 14 durch radiales Verstellen der Führungsarme 32, 34 stufenlos eingestellt werden, wobei dabei die benachbarten und sich jeweils überlappenden Folien 16 in- bzw. auseinander gleiten. Neben den Führungsarmen 32 stützen sich die Foliensegmente gegenseitig an den Überlappungsabschnitten 20. Die maximale Weitenverstellung wird durch die Anzahl der Folien 16 und deren jeweilige Länge bestimmt und ist nur bis zu einem Durchmesser möglich, bei dem sich benachbarte Folien 16 noch überlappen. Die Grenze für die Durchmesservergrößerung ist spätestens dann erreicht, wenn die Metallfoliensegmente keine
Führung mehr untereinander haben (siehe Fig. 11).

Wenn dagegen die Weite der Führungsarme 32, 34 verringert wird, wandern die Aufnahmen 54, 56 radial nach innen und die darin eingespannten Folien werden weiter nach innen gedrückt und noch mehr gebogen. Die einander überlappenden Folien 16 gleiten noch mehr ineinander und die Überlappungsabschnitte 20 werden noch größer.

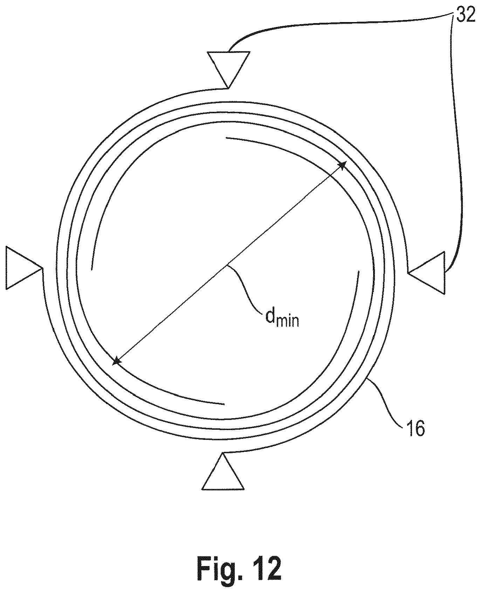

Auch wenn sich Folien 16 nahezu beliebig nach innen aufrollen und ineinander schieben lassen, so ist eine gewisse Grenze erreicht, wenn jede Folie 16 im Wesentlichen um 360° gebogen worden ist und an jeder Stelle 4 Lagen übereinander liegender Foliensegmente16 vorliegen (siehe Fig. 12).

Für die Weitenverstellung der Folien 16 ist es notwendig, dass diese in Umfangsrichtung aus- und ineinander gleiten können und so einen bestimmten Freiheitsgrad in Umfangsrichtung benötigen. Um jedoch ein Herausrutschen oder -fallen der Folien aus den Führungsarmen in axialer Richtung zu verhindern oder um zu verhindern, dass die Folien in Umfangsrichtung immer gleich beanstandet bleiben, kann jeweils ein Ende der Metallfoliebänder 16 oder -segmente an dem entsprechenden Führungsarm 32, 34 angeklebt, angelötet oder anderweitig befestigt sein, während das andere Ende frei beweglich bleibt.

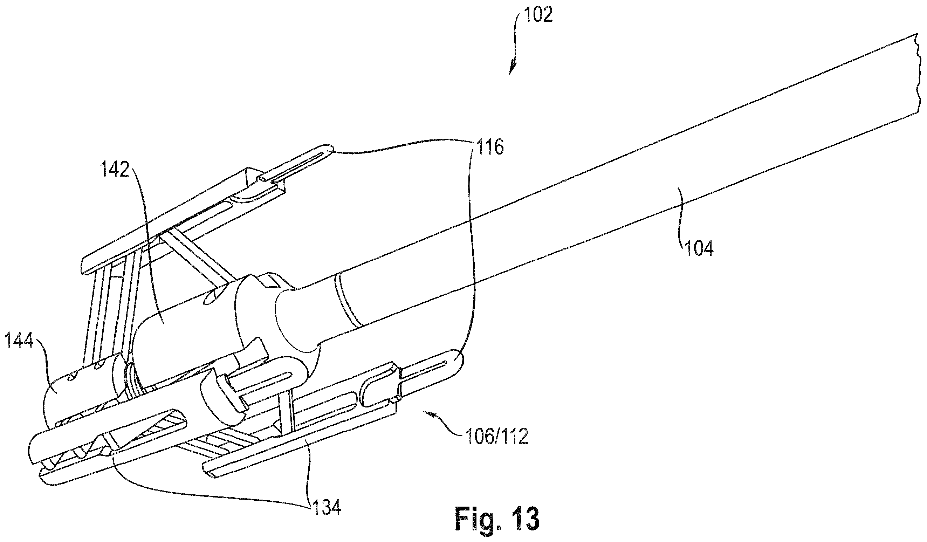

Die Fig. 13 zeigt einen distalen Abschnitt eines Schneidinstruments 102 gemäß einer dritten Ausführungsform der Erfindung. Dieses Schneidinstrument 102 ist im Aufbau und seiner Mechanik sehr ähnlich zur den ersten beiden Ausführungsformen, weist jedoch am distalen Ende eines Werkzeugschafts 104 keine Stempel-Matrizen-Anordnung, sondern ein ebenfalls über einen Stellmechanismus weitenverstellbares Schneidwerkzeug 112 auf, welches um die Instrumentenschaftachse drehbar ist. Die Schneide wird nicht durch eine Folienklinge, sondern durch einzelne Schneidklingen 116 gebildet, die jeweils an einem distalen axialen Ende von Tragarmen 134 lösbar, z.B. in einen Axialschlitz eingesteckt, oder fest befestigt sind und sich in axialer bzw. schaftparallelen Richtung erstrecken und in beiden Umfangsrichtungen mit Schneiden 114 versehen sind. Die Klingen 116 befinden sich wiederum um den Abstand x radial weiter innen als die Außenflächen der Tragarme 134. Die Schneidklingen 116 erstrecken sich entgegen der Einführrichtung des Instruments 102, um nicht versehentlich als Bajonett zu wirken und beim Einführen Gewebe zu verletzen. Durch Drehen des Schneidwerkzeugs 110 in beliebige Richtung führen die drei Schneidklingen 116 einen zirkulären Schnitt durch. Die Tragarme 134 sind wie bei den ersten beiden Ausführungsformen über eine Stellmechanismus parallel zur Schaftachse verschiebbar, wodurch sich der Schneiddurchmesser der Schneidklingen 116 einstellen lässt. Der Stellmechanismus ist im Wesentlichen identisch zu den ersten beiden Ausführungsformen, so dass sich auch hier über einen nicht gezeigten Drehknopf am distalen Ende des Instrumentenschafts eine Schafteinheit mit einer oben detailliert beschriebenen Parallelogramm-Mechanik betätigbar ist. Ein Unterschied zu den ersten beiden Ausführungsformen liegt darin, dass zwischen den relativ zueinander verschiebaren und die Weitenverstellung bewirkenden Schäfte, 142, 144 eine Feder 143 angeordnet ist, welche die beiden Schäfte 142, 144 in eine Verstellrichtung vorspannen. Alternativ kann die Weitenverstellung über ein Gewinde erfolgen.

Die Funktionsweise des Instruments 102 ist sehr ähnlich zum Instrument 2. Zum Einführen des Instruments 102 werden die Tragarme 134 und die Schneidklingen 116 so nahe wie möglich an den Werkzeugschaft 104 herangezogen. Dann wird das Instrument 102 transapikal eingeführt und das Schneidwerkzeug 112 mittig durch die Aortenklappenöffnung geführt. Anschließend werden die Tragarme 134 durch Betätigen des Stellmechanismus soweit aufgespreizt, bis sich die Tragarme 134 an der Aorteninnenwand abstützen. In einer Schraubbewegung, d.h. in einer zum distalen Ende des Instrumentenschafts hin gerichtete überlagerte axiale und rotatorische Bewegung resezieren die Klingen 116 die Aortenklappe. Diese Schnittbewegung kann über ein am distalen Ende des Instrumentenschafts vorgesehenes Handstück oder motorisch erfolgen.

Die vorliegende Erfindung wurde anhand bevorzugter Ausführungsformen dargestellt, sie ist jedoch nicht auf diese beschränkt.

So kann die Anzahl der Folienführungen, Foliensegmente und Schneidklingen variiert werden. Jedoch macht es Sinn, die Anzahl so groß wie möglich zu wählen, damit eine gute Führung der Metallfoliensegmente gewährleistet wird.

Wie oben beschrieben, fahren Stempel und Matrize einen bestimmten Weg ineinander und schneiden so das dazwischen liegende Gewebe ab. Es ist aber auch vorstellbar, dass sich die Aufnahmen von Stempel und Matrize nicht unterscheiden und beide Ringschneiden den gleichen Durchmesser haben. Ferner kann die Schneideinheit auch wie eine Knochenstanze arbeiten, wobei hier nur eines der beiden Werkzeuge eine Schneide aufweist und das andere Werkzeug lediglich als Amboss dient.

Es ist auch vorstellbar, dass über den Folienführungen ein engmaschiges, elastisches Netz aufgesetzt ist, wie dies bereits in den Fig. 4 und Fig. 5 angedeutet ist. Dieses Netz macht die Verstellung der Stanzdurchmesser mit und bildet beim Stanzvorgang einen geschlossenen Raum, in dem die abgetrennten Aortenklappen aufgefangen und sicher geborgen werden können. Somit wird auch verhindert, dass Kalkpartikel in die Blutbahn gelangen. Durch das Netz wird beim Stanzen auch der Blutstrom aufrecht erhalten.

Bei der oben gezeigten Ausführungsform ist die Folienklinge lediglich radial außen geführt, da sich die Folie bzw. die Foliensegmente von sich abrollen wollen und radial nach außen drücken. Die Folien können jedoch auch zusätzlich innen geführt sein. In einer Variante ist die radial innere Führung nachführbar, um so einen sich ergebenden Spalt in der Folienführung ausgleichen zu können und ggf. die Folien für den Schneidvorgang zu verspannen.

In einer Variante kann der gesamte distale Abschnitt, auf dem das zweite Schneidwerkzeug sitzt, separat eingeführt werden und im intrakorporalen Rendezvous-Verfahren mit dem Instrument gekoppelt oder nach dem erfolgreichen Schneidvorgang über einen mit dem Handstück bedienbare Mechanik vom Instrument gelöst werden und anderweitig geborgen werden.

Bei einer weiteren Variante führen die beiden Schneidwerkzeuge, wenn sie über das Handstück zum Abschneiden der Aortenklappe zusammengeführt werden, nicht nur eine reine translatorische Bewegung, sondern eine kombinierte translatorische und rotatorische Bewegung bzw. Schraubbewegung durch.

### Bezugszeichenliste

- 2, 102: Schneidinstrument
- 4, 104: Werkzeugschaft
- 6: Schneideinheit
- 8: Handstück
- 10: Schneidwerkzeug (Stempel)
- 12, 112: Schneidwerkzeug (Matrize)
- 14: Schneide
- 16: Folie, Foliensegmente
- 18: Kante
- 20: Überlappungsabschnitte
- 22: Handgriff
- 24: Handgriff
- 26: Feder
- 28: erste Schafteinheit
- 30: zweite Schafteinheit
- 32: erste Führungsarme
- 34, 134: zweite Führungsarme
- 36: Gelenkarme
- 38: äußerer Hohlschaft
- 40: innerer Hohlschaft
- 42, 142: äußerer Hohlschaft
- 143: Feder
- 44, 144: innerer Schaft
- 46: Drehknopf
- 48: Axialstift
- 50: Drehteil
- 52: Schaftendstück
- 54: Aufnahme
- 56: Aufnahme
- 58: Aortenwandung

## Patentansprüche

1. Chirurgisches Schneidinstrument (2; 102) zur Aortenklappenresektion, mit einer am distalen Ende eines Werkzeugschafts (4; 104) angeordneten Schneideinheit (6; 106) mit zumindest einem mechanischen Schneidelement (14; 116) zum Durchführen eines zirkulären Schnitts,
**dadurch gekennzeichnet, dass**
das zumindest eine Schneidelement (14; 116) über einen radial bewegbaren Stellmechanismus an unterschiedliche Aortendurchmesser anpassbar ist, wobei der Stellmechanismus derart ausgebildet ist, dass einer oder mehrere in Umfangsrichtung verteilte Tragarme bzw. Führungsarme (32, 34; 134), welche das zumindest eine Schneidelement (14; 116) tragen oder führen, parallel zu der Werkzeugschaftachse ausstellbar und einziehbar sind, so dass das zumindest eine Schneidelement (14; 116) über einen gesamten Einstellbereich stets achsparallel bzw. parallel zur Werkzeugschaftachse ausgerichtet bleibt.

2. Chirurgisches Schneidinstrument (2; 102) nach Anspruch 1, **dadurch gekennzeichnet, dass** das zumindest eine Schneidelement (14; 116) über den radial bewegbaren Stellmechanismus stufenlos an unterschiedliche Aortendurchmesser anpassbar ist.

3. Chirurgisches Schneidinstrument (2) nach Anspruch 1 oder 2, **gekennzeichnet durch** einen am proximalen Ende des Werkzeugschafts (4, 104) angeordneten Werkzeuggriff (52) mit einer manuell betätigbaren Handhabe (46), die mit dem Stellmechanismus gekoppelt ist, um das in Schneidfunktionsstellung bereits sich befindende Schneidelement (14, 116) manuell weiten zu verstellen.

4. Chirurgisches Schneidinstrument (2) nach einem der Ansprüche 1 bis 3, **dadurch gekennzeichnet, dass**
die Schneideinheit (6) durch eine zumindest in axialer Richtung relativ zueinander bewegbare Stempel-Matrizen-Anordnung (10, 12) gebildet wird, wobei eine Stempeleinheit (10) und/oder eine Matrizeneinheit (12) eine dünne, biegeelastische, ringförmig gebogene und sich in Umfangsrichtung spiralförmig überlappende Folie (16) aufweist, wobei die sich hieraus ergebenden Folienschichten plan aneinander liegen, derart, dass eine Stirnkante der Folie (16) in aufspiraltem Zustand eine einzige, gemeinsame, im Wesentlichen ringförmige Schneide (14) bildet, deren Durchmesser durch Ein- und Aufrollen der spiralförmig aufgewickelten Folie (16) stufenlos an unterschiedliche Aortendurchmessern anpassbar ist.

5. Chirurgisches Schneidinstrument (2) nach einem der Ansprüche 1 bis 3, **dadurch gekennzeichnet, dass**
die Schneideinheit (6) durch eine zumindest axial relativ zueinander bewegbare Stempel-Matrizen-Anordnung (10, 12) gebildet wird, wobei eine Stempeleinheit (10) und/oder eine Matrizeneinheit (12) eine ringförmige Anordnung mehrerer gebogener, sich in Umfangsrichtung überlappender, dünner sowie biegeelastischer Folien (16) aufweist, die plan aneinander liegen, derart, dass deren Kanten eine einzige, gemeinsame, im Wesentlichen ringförmige Schneide (14) bilden, deren Durchmesser durch Relativverschiebung der Folien (16) in Umfangsrichtung stufenlos an unterschiedliche Aortendurchmesser anpassbar ist.

6. Chirurgisches Schneidinstrument (2) nach Anspruch 4 oder 5, **dadurch gekennzeichnet, dass** die Folie (16) oder die Folien (16) aus Metall oder NiTiNol sind.

7. Chirurgisches Schneidinstrument (2) nach einem der Ansprüche 4 bis 6, **dadurch gekennzeichnet, dass**
die zumindest eine Folie (16) oder die Mehrzahl von Folien in radialer Richtung lediglich außen durch Folienführungen (32, 34) abgestützt ist/sind, die über den Stellmechanismus radial verstellbar sind, und die zumindest eine gebogene Folie oder die Mehrzahl von Folien (16) aufgrund ihrer Eigenflexibilität selbstständig einer Weitenverstellung radial nach außen folgt/folgen.

8. Chirurgisches Schneidinstrument (2) nach einem der Ansprüche 4 bis 7, **dadurch gekennzeichnet, dass**
die (Gesamt-) Länge der zumindest einen Folie (16) oder der Mehrzahl von Folien (16) insgesamt länger als der Umfang des größten einstellbaren Schneiddurchmessers ist.

9. Chirurgisches Schneidinstrument (2) nach einem der Ansprüche 4 bis 8, **dadurch gekennzeichnet, dass**
zumindest ein Folienende der zumindest einen Folie (16) abgeschrägt bzw. spitz zulaufend ist.

10. Chirurgisches Schneidinstrument (2) nach Anspruch 7, **dadurch gekennzeichnet, dass**
die Anzahl der Folien (16) der Anzahl der Folienführungen (32, 34) entspricht.

11. Chirurgisches Schneidinstrument (2) nach Anspruch 7 oder 10, **dadurch gekennzeichnet, dass**
sich der durch die Folienführungen (32) der Stempeleinheit (10) definierte Foliendurchmesser und der durch die Folienführungen (34) der Matrizeneinheit (12) definierte Foliendurchmesser so unterscheiden, dass sich die beiden Schneiden (14', 14") aneinander vorbeibewegende bzw. scheren.

12. Chirurgisches Schneidinstrument (2) nach einem der Ansprüche 7, 10 oder 11, **dadurch gekennzeichnet, dass**
über die Folienführungen (32, 34) ein engmaschiges, elastisches Netz aufgesetzt ist, welches der Weitenverstellung folgt und beim Stanzvorgang einen geschlossenen Raum bildet, in dem die abgetrennten Aortenklappen aufgefangen und sicher geborgen werden können.

13. Chirurgisches Schneidinstrument (2) nach einem der Ansprüche 4 bis 12, **dadurch gekennzeichnet, dass**
die Stempeleinheit (10) und die Matrizeneinheit (12) lösbar miteinander gekoppelt sind und der distale Teil von beiden mit bzw. vom Werkzeugschaft (4) gekoppelt bzw. entkoppelt werden kann.

14. Chirurgisches Schneidinstrument (102) nach einem der Ansprüche 1 bis 3, **dadurch gekennzeichnet, dass**
die Schneideinheit (106) durch ein um den Werkzeugschaft (104) drehbar gelagertes Schneidwerkzeug (112) mit zumindest einer Schneidklinge (116) gebildet wird, deren radialer Abstand zur Drehachse über den Stellmechanismus stufenlos an unterschiedliche Aortendurchmessern anpassbar ist.

15. Chirurgisches Schneidinstrument (2, 102) nach einem der Ansprüche 1 bis 14, **dadurch gekennzeichnet, dass**
der Stellmechanismus die mit einander gekoppelten, axial ausgerichteten Tragarme oder Führungsarme (32, 34; 134), welche als Folienführungen (32, 34) oder Schneidklingenträger (134) dienen, mittels einer Parallelogramm-Mechanik parallel zur Werkzeugschaftachse verschieben kann.

## Claims

1. A surgical cutting instrument (2; 102) for aortic valve resection, comprising a cutting unit (6; 106) arranged on the distal end of a tool shaft (4; 104) and comprising at least one mechanical cutting element (14; 116) for making a circular incision,
**characterized in that**
the at least one cutting element (14; 116) can be adapted to different aortic diameters by means of a radially movable actuating mechanism, wherein the actuating mechanism is designed such that one or more supporting arms or guiding arms (32, 34; 134) distributed in the circumferential direction and supporting or guiding the at least one cutting element (14; 116) can be expanded and retracted parallel to the tool shaft axis, so that the at least one cutting element (14; 116) always remains axially parallel or in parallel alignment relative to the tool shaft axis throughout the entire adjustment range.

2. The surgical cutting instrument (2; 102) according to claim 1, **characterized in that** the at least one cutting element (14; 116) can be continuously adapted to different aortic diameters by means of the radially movable actuating mechanism.

3. The surgical cutting instrument (2) according to claim 1 or 2, **characterized by** a tool handle (52) arranged on the proximal end of the tool shaft (4; 104) and comprising a manually actuatable handle piece (46) which is coupled to the actuating mechanism to manually adjust the width of the cutting element (14; 116) which is already in the functional cutting position.

4. The surgical cutting instrument (2) according to one of claims 1 to 3, **characterized in that**
the cutting unit (6) is formed by a punch-die assembly (10, 12) being movable relative to each other at least in the axial direction, a punch unit (10) and/or a die unit (12) comprising a thin, elastically bendable, annularly bent foil (16) which overlaps itself in the circumferential direction in spiral fashion, the foil layers resulting therefrom having a mutual flat contact such that a front edge of the foil (16) in a spiraled state forms a single, common and essentially annular blade (14) whose diameter can be continuously adapted to different aortic diameters by curling up or unrolling the spirally coiled foil (16).

5. The surgical cutting instrument (2) according to one of claims 1 to 3, **characterized in that**
the cutting unit (6) is formed by a punch-die assembly (10, 12) being movable relative to each other at least in the axial direction, a punch unit (10) and/or a die unit (12) comprising an annular assembly of several bent, thin and elastically bendable foils (16) which overlap in the circumferential direction and have a mutual flat contact such that their edges form a single, common and essentially annular blade (14) whose diameter can be continuously adapted to different aortic diameters by a relative movement of the foils (16) in the circumferential direction.

6. The surgical cutting instrument (2) according to claim 4 or 5, **characterized in that**
the foil (16) or foils (16) are made of metal or Nitinol.

7. The surgical cutting instrument (2) according to one of claims 4 to 6, **characterized in that**
the at least one foil (16) or the plurality of foils is/are supported by foil guides (32, 34) in the radial direction only on the outside, wherein the foil guides can be radially adjusted via the actuating mechanism, and the at least one bent foil or the plurality of foils (16) automatically follow a width adjustment radially outwards due to their inherent elasticity.

8. The surgical cutting instrument (2) according to one of claims 4 to 7, **characterized in that**
the (overall) length of the at least one foil (16) or the plurality of foils (16) is as a whole longer than the circumference of the maximally adjustable cutting diameter.

9. The surgical cutting instrument (2) according to one of claims 4 to 8, **characterized in that**
at least one foil end of the at least one foil (16) is slanted or tapered.

10. The surgical cutting instrument (2) according to claim 7, **characterized in that**
the number of the foils (16) is equal to the number of the foil guides (32, 34).

11. The surgical cutting instrument (2) according to claim 7 or 10, **characterized in that**
the foil diameter defined by the foil guides (32) of the punch unit (10) and the foil diameter defined by the foil guides (34) of the die unit (12) differ such that the two blades (14', 14") move past each other or perform a shear movement.

12. The surgical cutting instrument (2) according to one of claims 7, 10, or 11, **characterized in that**
the foil guides (32, 34) set up a fine-meshed, elastic net which follows the width adjustment and forms a closed space during the punching process, in which the severed aortic valves can be collected and safely recovered.

13. The surgical cutting instrument (2) according to one of claims 4 to 12, **characterized in that**
the punch unit (10) and the die unit (12) are detachably coupled to each other and the distal part of them can be coupled to or uncoupled from the tool shaft (4).

14. The surgical cutting instrument (102) according to one of claims 1 to 3, **characterized in that**
the cutting unit (106) is formed by a cutting tool (112) which is rotatably supported around the tool shaft (104) and comprises at least one cutting blade (116) whose radial distance to the rotational axis can be continuously adjusted to different aortic diameters via the actuating mechanism.

15. The surgical cutting instrument (2, 102) according to one of claims 1 to 14, **characterized in that**
the supporting arms or guiding arms (32, 34; 134) which are coupled to each other and axially orientated and which serve as foil guides (32, 34) or a cutting blade carrier (134), can be shifted parallel to the tool shaft axis by the actuating mechanism by means of a parallelogram-type mechanic system.

## Revendications

1. Instrument chirurgical de coupe (2 ; 102) destiné à la résection de valve aortique, avec une unité de coupe (6 ; 106) agencée à l'extrémité distale d'une tige d'outil (4; 104) avec au moins un élément de coupe mécanique (14; 116) pour la réalisation d'une coupe circulaire,
**caractérisé en ce que** l'au moins un élément de coupe (14 ; 116) peut être adapté par l'intermédiaire d'un mécanisme de réglage mobile radialement à différents diamètres d'aorte,
dans lequel le mécanisme de réglage est réalisé de telle sorte qu'un ou plusieurs bras porteurs ou de guidage (32, 34 ; 134) qui sont répartis en direction circonférentielle et qui portent ou guident l'au moins un élément de coupe (14 ; 116) peuvent être sortis ou rentrés parallèlement à l'axe de tige d'outil de telle sorte que l'au moins un élément de coupe (14 ; 116) reste constamment orienté parallèlement à l'axe ou parallèlement à l'axe de tige d'outil sur toute une plage de réglage.

2. Instrument chirurgical de coupe (2 ; 102) selon la revendication 1, **caractérisé en ce que**, par l'intermédiaire du mécanisme de réglage mobile radialement, l'au moins un élément de coupe (14; 116) peut être adapté progressivement à différents diamètres d'aorte.

3. Instrument chirurgical de coupe (2) selon la revendication 1 ou 2, **caractérisé par** une poignée d'outil (52) agencée à l'extrémité proximale de la tige d'outil (4, 104) et munie d'une manette (46) actionnable manuellement qui est couplée au mécanisme de réglage pour continuer à régler manuellement l'élément de coupe (14, 116) qui se trouve déjà dans la position fonctionnelle de coupe.

4. Instrument chirurgical de coupe (2) selon l'une des revendications 1 à 3, **caractérisé en ce que** l'unité de coupe (6) est formée par un dispositif de poinçon et matrice (10, 12) mobiles l'un par rapport à l'autre au moins en direction axiale, une unité de poinçon (10) et/ou une unité de matrice (12) comportant une feuille (16) mince, souple, courbée en forme d'anneau et se chevauchant en spirale dans la direction circonférentielle, les couches de feuille résultantes se retrouvant à plat les unes à côté des autres de telle sorte qu'une arête frontale de la feuille (16) enroulée en spirale forme un seul tranchant (14), commun et globalement annulaire, dont le diamètre peut être adapté progressivement à différents diamètres d'aorte par l'enroulement et le déroulement de la feuille (16) enroulée en spirale.

5. Instrument chirurgical de coupe (2) selon l'une des revendications 1 à 3, **caractérisé en ce que** l'unité de coupe (6) est formée par au moins un dispositif de poinçon et matrice (10, 12) mobiles l'un par rapport à l'autre au moins en direction axiale, une unité de poinçon (10) et/ou une unité de matrice (12) comportant un agencement annulaire de plusieurs feuilles (16) minces, souples, courbées et se chevauchant dans la direction circonférentielle qui sont à plat les unes à côté des autres de telle sorte que leurs arêtes forment un seul tranchant (14), commun et globalement annulaire, dont le diamètre peut être adapté progressivement à différents diamètres d'aorte par le déplacement relatif des feuilles (16) dans la direction circonférentielle.

6. Instrument chirurgical de coupe (2) selon la revendication 4 ou 5, **caractérisé en ce que** la feuille (16) ou les feuilles (16) sont en métal ou en Nitinol.

7. Instrument chirurgical de coupe (2) selon l'une des revendications 4 à 6, **caractérisé en ce que** l'au moins une feuille (16) ou la multiplicité de feuilles sont soutenues en direction radiale simplement à l'extérieur par des guides de feuille (32, 34) qui sont réglables radialement par l'intermédiaire du mécanisme de réglage et **en ce que** l'au moins une feuille courbée ou la multiplicité de feuilles (16) suivent automatiquement un réglage de largeur radialement vers l'extérieur en raison de leur flexibilité propre.

8. Instrument chirurgical de coupe (2) selon l'une des revendications 4 à 7, **caractérisé en ce que** la longueur (totale) de l'au moins une feuille (16) ou de la multiplicité de feuilles (16) est globalement plus longue que la circonférence du plus grand diamètre de coupe réglable.

9. Instrument chirurgical de coupe (2) selon l'une des revendications 4 à 8, **caractérisé en ce qu'**au moins une extrémité de feuille de l'au moins une feuille (16) est biseautée ou se termine en pointe.

10. Instrument chirurgical de coupe (2) selon la revendication 7, **caractérisé en ce que** le nombre de feuilles (16) correspond au nombre de guides de feuille (32, 34).

11. Instrument chirurgical de coupe (2) selon la revendication 7 ou 10, **caractérisé en ce que** le diamètre de feuille défini par les guides de feuille (32) de l'unité de poinçon (10) et le diamètre de feuille défini par les guides de feuille (34) de l'unité de matrice (12) se distinguent de telle sorte que les deux tranchants (14', 14") se déplacent l'un devant l'autre ou se cisaillent.

12. Instrument chirurgical de coupe (2) selon l'une des revendications 7, 10 ou 11, **caractérisé en ce qu'**un réseau élastique, à mailles étroites, est défini par l'intermédiaire des guides de feuille (32, 34), lequel réseau suit le réglage de largeur et forme lors de l'opération de découpage un espace fermé dans lequel les valves aortiques peuvent être attrapées et dégagées de manière sûre.

13. Instrument chirurgical de coupe (2) selon l'une des revendications 4 à 12, **caractérisé en ce que** l'unité de poinçon (10) et l'unité de matrice (12) sont couplées l'une à l'autre de manière amovible et la partie distale des deux peut être couplée ou découplée de la tige d'outil (4).

14. Instrument chirurgical de coupe (102) selon l'une des revendications 1 à 3, **caractérisé en ce que** l'unité de coupe (106) est formée par un outil de coupe (112) logé rotatif autour de la tige d'outil (104) avec au moins une lame de coupe (116) dont la distance radiale à l'axe de rotation peut être adaptée progressivement à différents diamètres d'aorte par l'intermédiaire du mécanisme de réglage.

15. Instrument chirurgical de coupe (2, 102) selon l'une des revendications 1 à 14, **caractérisé en ce que** le mécanisme de réglage peut déplacer les bras porteurs ou bras de guidage (32, 34; 134), qui sont couplés ensemble et orientés de façon axiale et qui servent de guides de feuille (32, 34) ou de supports de lame de coupe (134), parallèlement à l'axe de tige d'outil au moyen d'un mécanisme parallélogramme.
